**Europäisches Patentamt**

**European Patent Office**

**Office européen des brevets**

(19)

(11) Publication number: **0 101 273**
**A2**

# EUROPEAN PATENT APPLICATION

(12)

(21) Application number: **83304549.5**

(22) Date of filing: **05.08.83**

(51) Int. Cl.³: **C 12 N 5/00**
**C 12 N 11/08**

(30) Priority: **06.08.82 US 405991**

(43) Date of publication of application:
**22.02.84 Bulletin 84/8**

(84) Designated Contracting States:
**AT BE CH DE FR GB IT LI LU NL SE**

(71) Applicant: **THE BOARD OF TRUSTEES OF THE LELAND STANFORD JUNIOR UNIVERSITY**
Encina 6-930, Stanford University
Stanford, California 94305(US)

(72) Inventor: **Robertson, Channing R.**
1089 Vernier Place
Stanford California 94305(US)

(72) Inventor: **Cohen, Stanley N.**
271 Gabarda Way
Portola Valley California 94025(US)

(72) Inventor: **Matin, Abdul**
690 Coronado Avenue
Stanford California 94305(US)

(72) Inventor: **Inloes, Douglas S.**
818 South Brentwood Boulevard Apartment No. 3a
Clayton Missouri 63105(US)

(72) Inventor: **Taylor, Dean**
400 Hillside Road
King of Prussia Pennsylvania 19406(US)

(72) Inventor: **Michaels, Alan S.**
111 Barrow Street Apartment No. 5c
New York New York 10014(US)

(74) Representative: **Allard, Susan Joyce et al,**
BOULT, WADE & TENNANT 27 Furnival street
London EC4A 1PQ(GB)

(54) Production of biological products using resting cells.

(57) A method of controlling cell growth and production of cell products is provided comprising sequentially contacting cells with a growth medium and then with a non-growth medium in which desirable cell products are produced.

EP 0 101 273 A2

# PRODUCTION OF BIOLOGICAL PRODUCTS USING RESTING CELLS

The present invention is directed to methods and means for controlling cell growth and increasing the production of cell products in systems which utilize microorganisms to produce desirable cell products. In particular, the present invention is directed to methods and means for controlling cell growth and increasing production of cell products in hollow-fiber membrane microbiological cell culture reactors.

This invention was made under Government support under Grant Nos. AI08619, GM26355 and GM27241 awarded by the National Institute of Health. The Government has certain rights in this invention.

Recent advances in genetic engineering have made it possible to enhance the expression of products normally synthesized by intact cells. For example, the amplification of gene expression may be achieved by altering the regulatory sites which control the induction or repression of gene transcription or by increasing the number of gene copies in the cell.

By known techniques genetic material from foreign or synthetic sources may also be introduced into host cells. As a consequence of such manipulation biological products which are either natural or foreign to normal gene expression of a host may be produced at enhanced levels.

Production of usable biological products has been achieved by two general approaches:

1) Methods which generate the biological product in an environment external to the living cell, as in the one-step bioconversion of substrate to product by an immobilized enzyme, and

2) Methods in vivo which generate biological products under conditions normally encountered in an intracellular environment, e.g., alcohol production by yeast fermentation of sugar.

In the case of immobilized enzymes a disadvantage is that the efficiency of bioconversion may be adversely affected by the failure to maintain an appropriate environment for the enzyme. Also, the technique is complicated by the fact that many enzymes have complex requirements, which include necessary cofactors, and in some cases the need to be associated with specific membrane structures. Therefore, optimal enzyme activity may often be achieved only within narrow ranges of temperature, pH, and ionic strength. The production of biological products outside a living cell also suffers from the disadvantage that there is an irreversible loss in bioconversion activity.

In the case of products produced in vivo, several disadvantages are also encountered. Since the cells used for production of biological products are often cultured under growth conditions, mass transport of nutrients to and waste products away from the culture pose significant problems. While various types of batch and continuous type microbiological reactors have been developed in an attempt to overcome these difficulties, there remains a need to improve efficiency of production of derived products by the cells.

Some problems in the operation of microbiological reactors made from artificial capillaries are that environmental stresses result from growing cell cultures, such as product inhibition, reduced nutrients supply and waste product buildup which may decrease the efficiency of the reactors. In addition, growing and dividing cells may be undesirable in reactor configurations that provide for a specified amount of volume for cell containment.

A further problem in the use of growing cultures in various types of micro-biological reactors is the possibility of culture reversion due to contamination or mutation. Cultures of genetically engineered cells which generate either abnormally high amounts of a natural product or a biological product which is non-essential or foreign to the host organism are at a selective disadvantage, and may sometime be particularly vulnerable to culture reversion.

We have developed a method and mean for controlling cell growth and improving the production of cell products by using resting cells which are reversibly maintained in a viable and productive state.

In the method which we have developed biological products normally not transferred across the cell membrane and/or cell

wall may be produced intracellularly in an organism and recovered extracellularly by inducing alterations of the cell membrane and/or wall by either the growth phase environment or the non-growth phase environment or by a combination thereof.

Furthermore, in the method which we have developed biological products normally not transferred across the cell membrane and/or cell wall may be produced intracellularly in an organism and recovered extracellularly by the use of an organism selected for its ability to allow the transfer of such biological products across the cell membrane and/ or cell wall.

The method which we have developed also provides an environment which will maximize the total productivity and/or life span of cell lines which exhibit a finite number of cell divisions before culture death or genetic reversion in the case of cells whose native DNA has been altered by one or more techniques commonly known as genetic engineering.

Accordingly, the present invention provides a method of controlling cell growth and production of cell products comprising the steps of sequentially (a) contacting the cells with a growth medium whereby the cells grow and divide; and (b) contacting the cells with a non-growth medium whereby growth and division are decreased and production of the cell products is increased.

This invention is directed to methods and means whereby biological products may be produced by resting cells maintained in a non-growth environment. An advantage in using resting cells is that cells which are not actively growing and dividing have lower nutrient requirements and generate small amounts of waste products. This allows the creation of a quasi-steady state environment which decreases the stress on the culture. In addition, the problem of cell reversion is diminished since the non-growth environment according to the present invention affects not only the productive cell line but also contaminants and mutants. The non-growth environment also limits the invasion of cells into the fiber lumen in the case of hollow fiber microbiological reactors.

Non-growth environments include but are not limited to:

1) a culture medium which lacks a source of nitrogen, or other essential growth nutrient;

2) a culture medium containing inhibitors of DNA synthesis; and

3) a culture medium comprising a mutant strain or cell line in which DNA replication is sensitive to a controllable environmental condition, such as, temperature, pH, and ionic strength.

Non-growth may also be achieved by producing the desired biological product in mutants in which DNA synthesis may be suppressed by a non-permissive environment. This approach includes but is not limited to the use of temperature sensitive mutants in which DNA replication is inhibited at certain temperatures. Mutations which inhibit replication should be reversible and the mutation should permit maintenance on plasmid vectors. Chemical blocking of DNA synthesis may also be utilized for appropriate cultures by known DNA synthesis blocking agents such as, arylazopyrimidines and hydroxyurea.

The method according to the present invention comprises the following steps:

    a)    a growing phase during which cells actively grow and divide; and

    b)    a production phase during which the cells rest, as normally evidenced by non-existent or low rate of overall cell division in the culture. The production phase may be brought on by an aforementioned non-growth environment.

The steps a) and b) may be alternately repeated in order to maximize useful production from a particular cell culture.

Figure 1 is a schematic diagram of a hollow fiber microbiological reactor capable of being used in the practice of this invention.

Figure 2 is a detailed schematic diagram of a hollow fiber membrane reactor.

Figure 3 is a graph showing ethanol productivity as a function of culture time.

Figure 4 is a graph of a fermentation efficiency vs. culture time.

The method according to the present invention is useful in microbiological reactor systems in which a cell culture may be sequentially brought into contact with different culture media.

In particular the invention may be practiced by the use of any type of microbiological reactor, and by batch or continuous operation, or by combinations which include features of both batch and continuous operation.

An exemplary batch type reactor may be used whereby cells are continuously mixed in a liquid suspension for a period of time after which the desired biological product is retrieved from the liquid medium or from disrupted cells. The method according to the invention may be practiced by allowing cells to grow and divide in a growth environment followed by a change in the liquid medium to a medium which will maintain the culture in the non-growth production phase.

Another reactor useful in accordance with the present invention is a continuously stirred tank reactor known as a chemostat with both an inlet and an outlet for continuous nutrient supply and product removal. The invention may be practiced in a chemostat by initial use of a complete medium for the growth phase followed by a gradual or substantially instantaneous change to the non-growth environment of the production phase. The desired product may be retrieved either from the circulating liquid or, in the case of products sequestered intracellularly, from either the disruption of cells washed out of the reactor or the batch-wise disruption of all or part of the cell population. The cells may be recycled to the tank reactor for re-use.

Other types of microbiological reactors which are amenable to the practice of this invention as described above include concentric air lift fermentors, tubular loop fermentors, tower fermentors, fluidized-bed fermentors, pressure cycle fermentors, semi-batch fermentors, fermentors coupled to dialysis membranes, cells immobilized in or on a solid matrix including hollow fiber membranes, etc.

A particularly preferred class of reactors comprises hollow fiber microbiological reactors. Generally such hollow fibers may be isotropic or asymmetric hollow fiber membranes. Isotropic hollow-fibers have a uniform tight-mesh structure throughout the entire membrane thickness whereas asymmetric hollow-fibers have a thin microporous inner lumen wall supported generally by a concentric thick macroporous matrix.

A preferred class of hollow fibers may be constructed of polymeric materials which may be hydrophobic, hydrophilic, positively or negatively charged, or neutral or combinations thereof. Generally, the outside diameter of such fibers may be greater than 300 um and less than 1500 um. The inside diameter of such fibers may be about 25% of the total diameter, however, the range of this dimension may be as high as approximately 90% of the total diameter. The wall thickness of hollow fibers may be greater than 15 u and less than 500 u. Examples of such fibers include XM-50, PM-30, PW-10 and Vita-fiber, all made by Amicon Corporation, cellulose acetate (HFU) and silcone polycarbonate, hollow fibers made by Dow Chemical Corporation, Celgard made by Celanese Corporation and Biofiber 80/5 made by Bio-Rad Laboratories. Examples of an isotropic fiber are polypropylene isotropic fibers made by Ghia-Membrana.

A wide variety of microorganisms, such as bacteria, yeast, fungi, etc., or proliferative cells derived from normal tissue, tumors, hybridomas, etc., may be utilized in accordance with the present invention. These may be naturally occurring strains or lines or modified strains or lines by conjugation or other known genetic engineering techniques.

Since a wide range of prokaryotic or eukaryotic cells can be used to practice the invention the specific growth environments used in the growth phase will depend upon the particular type of cell being cultured. In this respect the choice of growth environment may be readily determined by those of ordinary skill in the art of growing that particular cell type or microorganism.

The choice of non-growth environment for the production phase of the invention is in a like manner dependent upon the cell type or microorganism used. Since it is desirable to alternate between growth and non-growth, product producing phases in the practice of the invention of the non-growth environment should not affect the ability of the cells to regenerate biological capacity when re-exposed to the growth environment.

Non-growth environment may be attained by limiting a necessary nutrient for cell growth or cell division where such an environment does not result in culture death. An exemplary non-growth medium of this type is one which is deficient in nitrogen. Also, non-growth may be achieved by use of inhibitors of DNA synthesis to prevent cell growth and division. In such cases it is desirable that the strains carry more than one mutation to reduce the likelihood that the cells will regain the ability to replicate as a result of mutational reversion. Temperature sensitive mutants may also be utilized whereby DNA replication is inhibited within a defined temperature range. Examples of temperature sensitive mutants are dnaA or dnaC mutants of E. coli K-12, such as CRT 461.1, and dnaA or dnaI mutants of Bacillus subtilis, such as, BD 355.

The biological products obtained from the practice of the invention include natural products such as excreted or non-excreted proteins. Examples include enzymes, polypeptides, hormones, lymphokines, antibiotics, toxins, immunoglobulins, amino acids, organic acids, alcohols, ketones, aldehydes, etc. In addition, biological products which result from the biological transformation of a substrate may be obtained by practicing this invention. Biological products produced by engineering synthetic genes into a cell line or microorganism may also be produced.

The methods according to the present invention may also be used to transform effluents from commercial plants such as chemical, sewage or water treatment plants by pouring such streams through microbiological reactors.

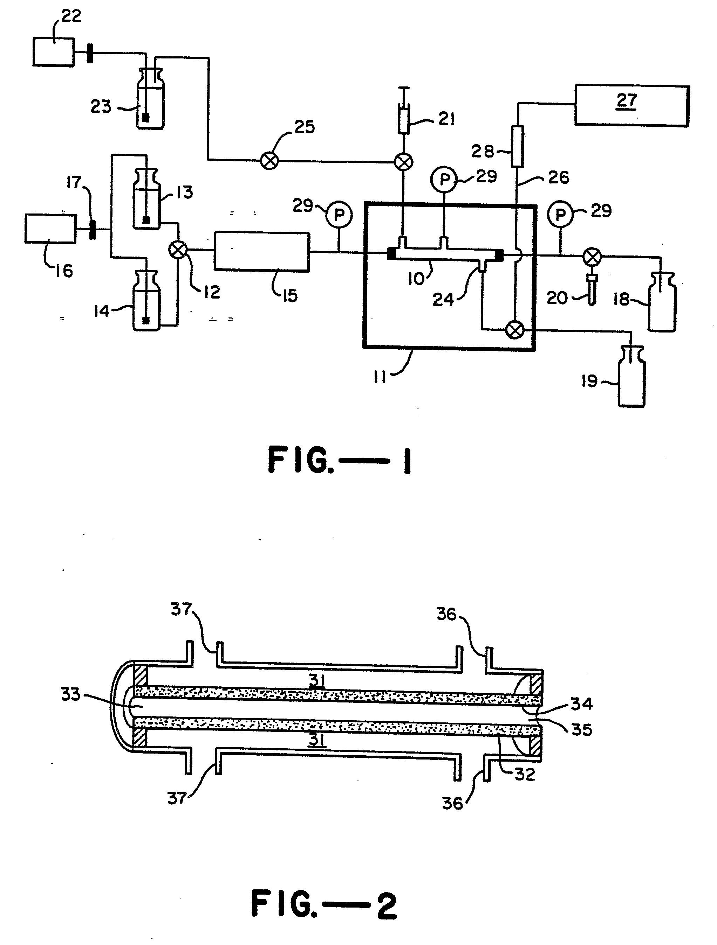

Figure 1 is a flow diagram of a microbiological reactor useful in accordance with the present invention. The microbiological reactor 10 containing hollow fiber membranes is located within gravity convection incubator 11 which maintains the environment of the reactor 10 at a constant temperature. By switching valve 12, either a growth medium contained in the vial 13 or a non-growth medium contained in vial 14 may be pumped through the reactors through pump 15, which is preferably a peristaltic pump. The media in vial 13 and 14 may be kept under inert atmosphere, preferably helium, or oxygen-containing atmosphere, as appropriate, which is bubbled into the vial from inert gas containing tank 16 which is filtered through a sterile filter 17. The liquid effluents from reactor 10 may be collected into reservoirs 18 and 19. Reservoir 18 is a liquid effluent from the lumen of the hollow fibers and reservoir 19 collects the liquid effluent from the shell space surrounding the lumen. Small samples of the lumen effluent may be taken off through sample tube 20. The reactor 10 may be inoculated with appropriate cell cultures through inoculating syringe 21. Following inoculation the same port may be used for flushing sterile, humidified air from tank 22 through humidifier 23 into reactor 10. The shell space outlet port 24 is located below the level of reactor 10 to allow extraneous fluid to drain from the fiber bundle, thus keeping the outer fiber surface in contact with the flowing gas stream. A micrometric capilary valve 25 is located upstream of the reactor 10 to control the air flow rate through the shell space. For measurement of the vapor phase concentrations, gas samples may be directed from the shell space effluent stream via line 26 into a gas chromatograph 27 through rotameter 28. Various pressures may be monitored by manometers located in various lines by manometers 29 located in various lines throughout the system.

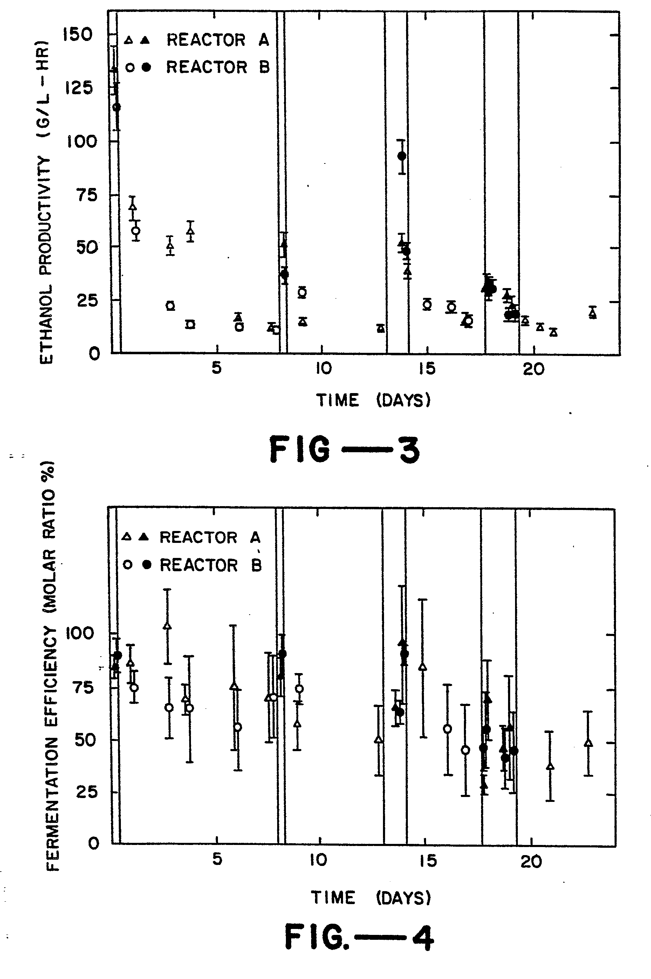

The design of a reactor with a single hollow fiber is schematically represented in Figure 2. Cell inoculum is introduced into the shell space 31 of reactor 30 through an inoculation port (not shown). Chemotactic microorganisms are capable of moving through large diameter pores on the fiber surface into the macro porous wall matrix 32 following inoculation. This movement into the wall matrix may be enhanced by pulling a slight suction on the fiber lumen 33 if necessary to establish non-motile microbes. The introduction of at least one viable cell into a macro pore will eventually lead to a dense cell packing as cells continue to grow and divide within the pore volume, essentially immobilizing and entrapping the actively growing cells within the wall matrix. Liquid phase nutrients are introduced into one end of the lumen and convectively flow through the lumen and penetrate the lumen membrane 34 into the macro porous region containing the cells. Cellular metabolic products, excreted into the extra cellular space within the fiber wall, may diffuse or convect radially in the reverse direction across the membrane 34 into the fiber lumen where they are subsequently swept from the fiber through an exit port of the fiber lumen 35. If required, continuous flushing of the shell space 31 with a gas stream through gas entry ports 36 will supply gaseous nutrients such as oxygen into the shell space, while simultaneously removing gaseous metabolic products such as carbon dioxide through gaseous exit ports 37. While Figure 2 has been described in connection with an asymmetric hollow fiber, the design of the reactor containing an isotropic hollow fiber is essentially the same as shown in Figure 2 except that the cells grow on the outer fiber surface 32 rather than within the fiber wall.

## Example 1

Two identical reactors similar to those illustrated in Figures 1 and 2, each containing a bundle of 30 asymmetric hollow-fiber membranes, were operated simultaneously under similar conditions. The particular membranes were polysulfone ultrafiltration hollow fibers (Amicon Corporation) having dimensions of 440 micrometers I.D. x 860 micrometers O.D. and a molecular weight cut off of 10,000 daltons. For each reactor, 30 such fibers were epoxied into a 25 centimeter by 10 millimeter I.D. glass shell. Both ends of each reactor were sealed with epoxy leaving only the fiber lumens open to flow. Three side ports in the glass shell, similar to those depicted in reactor 10 in Figure 1, were used to permit access to the reactor shell space for inoculation, air flushing, and pressure measurements. Both reactors passed a helium bubble-point leak test at 10 psi and were sterilized using an ethylene oxide gas mixture.

The complete growth medium contained the following components per liter of distilled water: glucose, 100 grams; yeast extract, 8.5grams; $NH_4Cl$, 1.32 grams; $CaCl_2$, 0.06 grams; $MgSO_4 \cdot 7H_2O$, 0.11 grams; potassium acid phthalate, 10.21 grams; and NaOH, 1.42 grams. The pH of the medium was 5.4.

The nitrogen deficient medium had the following components per liter of distilled water: glucose, 100 grams; $KH_2PO_4$, 1 gram; potassium acid phthalate, 10.21 grams; NaOH, 1.42 grams; $MgSO_4 \cdot 7H_2O$, 0.25 grams; $CaCl_2$, 0.06 grams; meso-inositol, 0.125 grams; mineral salts solution, 1.0 ml.; and vitamin solution, 1.0 ml. The mineral salt solution contained the following per 100 ml of distilled water: $ZnSO_4 \cdot 7H_2O$, 0.5 grams; $FeSO_4 \cdot 7H_2O$, 0.1 grams; $MnSO_4 H_2O$, 0.15 grams; $CuSO_4 \cdot 5H_2O$, 0.20 grams. The vitamin solution contained the following per 100 ml of distilled water: thiamine hydrochloride, 0.5 grams; pyridoxine hydrochloride, 0.625 grams; nicotinic acid, 0.5 grams; Ca-D-pathothenate, 0.625 grams; D-biotin, 0.0125 grams.

All media were sterilized by sterile filtration through a 0.22-um Millipore membrane. In order to prevent the blockage of the hollow fibers by gas bubbles, both growth and nitrogen deficient media were flushed with helium gas to remove dissolved air. The reactor was assembled as shown in Figure 1 and equilibrated with growth medium.

The yeast innoculum was an exponential-phase culture of S. cerevisiae (ATCC 4126) grown in growth medium, to a density $10^8$ cells per ml. The shell space, shown diagramatically in Figure 2, was inoculated with S. cerevisiae by means of the inoculum syringe shown in Figure 1. After one hour the shell space was drained and thereafter was flushed with humidified air at the rate of 20 ml per minute. Growth medium was perfused through the fiber lumen as diagrammed in Figure 2 for ten hours. A cell density of $10^9$-$10^{10}$ cells/ml was attained. The non-growth nitrogen deficient medium was thereafter perfused through the fiber lumen until the eighth day of the culture. At that time growth medium was perfused through the fiber lumen for eight hours. This was followed by a non-growth phase which lasted until the thirteenth day of culture, at which time a 24-hour growth phase was initiated, followed by a non-growth phase which ended at day 18. At that time a 40-hour growth phase was initiated followed by a final non-growth phase.

The growth phases are represented in Figures 3 and 4 by the shaded areas. As shown in Figure 3, the ethanol production rate during the initial growth phase was approximately 133 gm/1-hr. After exposure to a nitrogen deficient medium, however, the productivity steadily declined before stabilizing at a level between 10 and 15 gm/1-hr. after five days. Subsequent growth phases at eight, thirteen and eighteen days corrolate with a significant increases in ethanol productivity as shown in Figure 3. During the three periods of nitrogen deficiency the ethanol productivity always appeared to stabilize between 10 and 15 gm/l-hr.

Figure 4 is a plot of the fermentation efficiency as a function of time after inoculation. The fermentation efficiency of 100% occurs under the ideal conversion of one mole of glucose to two moles of ethanol and carbon dioxide. As can be seen from Figure 4, growth phases generally bring about an increase in the fermentation efficiency of a culture exposed to a non-growth medium.

## Example 2

Human choriocarcinoma cells are grown in a microbiological reactor on artificial capillaries according to the method of Knazek (Patent No. 3,821,087). After the desired cell density is achieved, the culture medium is replaced with the same medium containing methotrexate. When necessary, the complete growth medium without inhibitors of DNA synthesis replaces the non-growth medium to regenerate the capacity to produce human chorionic gonadotropin.

## Example 3

Bacillus subtilis (749/C), a mutant whose DNA replication can be inhibited by elevated temperature, is grown in a complete medium on artificial capillaries in a microbiological reactor. This organism excretes penicillinase at high rates. After the desired cell density is achieved, the temperature of the reactor and/or growth medium is raised to inhibit cell division and growth. Penicillinase is recovered from the perfusion medium during both growth and production phases.

Example 4

*Eschericha coli* K-12 (CRT 461.1), containing PBR 322, is a temperature sensitive mutant whose DNA synthesis is inhibited by elevated temperatures. The plasmid produces penicillinase which is normally retained intracellularly in the bacteria. The organism is grown in a chemostat in a complete growth medium to a desired cell density. The temperature of the chemostat is raised to inhibit further growth and cell division. Cells washed out of the chemostat are isolated and penicillinase is recovered from disrupted cells. The desired cell density within the chemostat is regenerated by reducing the temperature to allow cell growth and division.

CLAIMS :

1.    A method of controlling cell growth and production of cell products comprising the steps of sequentially (a)  contacting the cells with a growth medium whereby the cells grow and divide; and (b)  contacting the cells with a non-growth medium whereby growth and division are decreased and production of the cell products is increased.

2.    A method as claimed in Claim 1 wherein the non-growth medium is deficient in an essential nutrient for growth and/or cell division.

3.    A method as claimed in Claim 1 wherein the non-growth medium contains an inhibitor of DNA synthesis.

4.    A method as claimed in Claim 1 wherein the cells are temperature sensitive mutants whose DNA replication is selectively inhibited in a predetermined temperature range.

5.    A method as claimed in any one of the preceding claims wherein the steps are alternatively repeated.

6.    A method as claimed in any one of the preceding claims in which the cells are yeast cells immobilized on hollow fiber membranes.

7.    A method as claimed in Claim 6 wherein the non-growth medium lacks a source of nitrogen and the cell product is ethanol.

1/2

FIG.—1

FIG.—2

0101273

2/2

FIG — 3

FIG. — 4